# EUROPEAN PATENT APPLICATION

(11) **EP 4 767 955 A1**
(43) Date of publication of application: **01.07.2026**
(21) Application number: 26150073.0
(22) Date of filing: 02.01.2026
(51) Int. Cl.: A61B 17/32

(54) **TECHNOLOGIES FOR SECURING A TISSUE PAD TO A JAW CLAMP OF AN ENERGY-BASED SURGICAL INSTRUMENT**

(30) Priority: 31.12.2024 US 202463740955 P; 29.09.2025 US 202519343717
(71) Applicant: Cilag GmbH International, 6300 Zug (CH)
(72) Inventor: SHELTON, IV, Frederick E., Cincinnati, 45242 (US); ARONHALT, Jacqueline, Cincinnati, 45242 (US); MESSERLY, Jeffrey D., Cincinnati, 45242 (US)
(74) Representative: Carpmaels & Ransford LLP

(57) **Abstract**

Technologies for securing a tissue pad to a jaw clamp of an energy-based surgical instrument includes a surgical instrument having an end effector. The end effector includes an ultrasonic blade and a jaw clamp. The jaw clamp includes a metallic frame and a tissue pad attached to the metallic frame. The tissue pad of the jaw clamp has a minimum width that is greater than width of the ultrasonic blade. Additionally, the sidewalls of the tissue pad and the metallic frame may be beveled so as to retain the tissue pad in the metallic frame.

## Description

This application claims the benefit of and priority to U.S. Patent Application No. 63/740955, entitled "TECHNOLOGIES FOR SECURING A TISSUE PAD TO A JAW CLAMP OF AN ENERGY-BASED SURGICAL INSTRUMENT," which was filed on December 31, 2024, and which is incorporated herein by reference in its entirety.

### TECHNICAL FIELD

The present disclosure relates generally to energy-based surgical instruments and, more particularly, to harmonic and/or electrosurgical surgical instruments.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode may be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instruments may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to an aspect of the present disclosure, an end effector of a surgical instrument may include a jaw clamp and an ultrasonic blade. The jaw clamp may include a metallic frame and a tissue pad attached to the metallic frame. The tissue pad may be embodied as or otherwise include a polytetrafluoroethylene (PTFE) substrate. The ultrasonic blade may be configured to contact the tissue pad of the jaw clamp when the end effector is in a closed position. The metallic frame of the jaw clamp may include a pair of metallic sidewalls. Each metallic sidewall may include a tab that extends longitudinally along the corresponding metallic sidewall. The tissue pad may also include a pair of sidewalls. Each sidewall of the tissue pad may include a slot that extends longitudinally along the corresponding sidewall. A minimum width of the tissue pad is defined between the slots of the pair of sidewalls. The minimum width of the tissue pad may be greater than a width of the ultrasonic blade.

In some embodiments, each sidewall of the tissue pad may be embodied as a vertical sidewall and the tabs of the metallic sidewall extend laterally into the slots of the sidewalls. Additionally, in some embodiments, each sidewall of the tissue pad may include an upper tab that extends laterally outwardly from the tissue bad and a lower tab that extends laterally outwardly from the tissue pad to define the respective slot in the sidewall. In some embodiments, the metallic frame may include a distal end that wraps upwardly around a distal end of the tissue pad. Additionally, in some embodiments, the tissue pad may further include a top surface that is contacted by the ultrasonic blade and a bottom surface opposite the top surface. Each of the top surface and the bottom surface may have equal widths.

According to another aspect of the present disclosure, an end effector of a surgical instrument may include a jaw clamp having a metallic frame and a tissue pad attached to the metallic frame. The tissue pad may include a polytetrafluoroethylene (PTFE) substrate. Additionally, the metallic frame of the jaw clamp may include a pair of beveled metallic sidewalls, and the tissue pad may also include a pair of beveled sidewalls configured to mate with the beveled sidewalls of the metallic frame to retain the tissue pad in the metallic frame.

In some embodiments, the beveled sidewalls of the metallic frame may be beveled inwardly. In such embodiments, the beveled sidewalls of the metallic frame may be beveled outwardly. Additionally, the metallic frame may include a distal end that wraps upwardly around a distal end of the tissue pad.

In some embodiments, the end effector may further include an ultrasonic blade configured to contact the tissue pad when the end effector is in a closed position. In such embodiments, an uppermost point of each beveled sidewall may define a minimum width of the tissue pad. The minimum width of the tissue pad is greater than a width of the ultrasonic blade. Additionally, in such embodiments, the tissue pad may further include a top surface that is contacted by the ultrasonic blade and a bottom surface opposite the top surface. Each of the top surface and the bottom surface may have different widths. For example, the top surface of the tissue pad may have a width that is greater that the width of the bottom surface of the tissue pad. Alternatively, the top surface of the tissue pad may have a width that is less than the width of the bottom surface of the tissue pad. Additionally, in some embodiments, the top surface ma include a pair of lateral tabs that extend laterally over the corresponding beveled sidewalls of the tissue pad. Additionally or alternatively, the metallic frame may include a distal end that wraps upwardly around a distal end of the tissue pad.

According to a further aspect of the present disclosure, a surgical instrument may include an end effector having a jaw clamp and an ultrasonic blade. The jaw clamp may include a metallic frame and a tissue pad attached to the metallic frame. The tissue pad of the jaw clamp may have a minimum width that is greater than a width of the ultrasonic blade.

In some embodiments, the tissue pad may include a polytetrafluoroethylene (PTFE) substrate. Additionally, in some embodiments, the metallic frame of the jaw clamp may include a pair of metallic sidewalls. Each metallic sidewall may include a tab extending longitudinally along the corresponding metallic sidewall. Additionally, the tissue pad may include a pair of sidewalls and each sidewall may include a slot extending longitudinally along the corresponding sidewall. The tabs of the metallic sidewalls may extend into the corresponding slots of the tissue pad.

In some embodiments, the metallic frame may include a pair of beveled sidewalls. In such embodiments, the tissue pad may also include a pair of beveled sidewalls configured to mate with the beveled sidewalls of the metallic frame to retain the tissue pad in the metallic frame. Additionally, in such embodiments, the beveled sidewalls of the metallic frame may be beveled inwardly, and the beveled sidewalls of the metallic frame may be beveled outwardly. Furthermore, in some embodiments, the metallic frame includes a distal end that wraps upwardly around a distal end of the tissue pad.

### BRIEF DESCRIPTION OF THE DRAWINGS

The detailed description particularly refers to the following figures, in which:
FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a perspective view of a jaw clamp of the surgical instrument of FIG. 2 including a tissue pad secured to a frame of the jaw clamp;
FIG. 9 is a cross-sectional view of a prior art jaw clamp denoting possible pad failure areas during functional use;
FIG. 10 is a cross-sectional view of an embodiment of the jaw clamp of FIG. 8 taken generally along the cross-sectional lines A-A;
FIG. 11 is a cross-sectional view of another embodiment of the jaw clamp of FIG. 8 taken generally along the cross-sectional lines A-A;
FIG. 12 is a cross-sectional view of another embodiment of the jaw clamp of FIG. 8 taken generally along the cross-sectional lines A-A;
FIG. 13 is a cross-sectional view of an embodiment of the jaw clamp of FIG. 8 taken generally along the cross-sectional lines B-B;
FIG. 14 is a cross-sectional view of another embodiment of the jaw clamp of FIG. 8 taken generally along the cross-sectional lines B-B; and
FIG. 15 is a cross-sectional view of another embodiment of the jaw clamp of FIG. 8 taken generally along the cross-sectional lines B-B.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

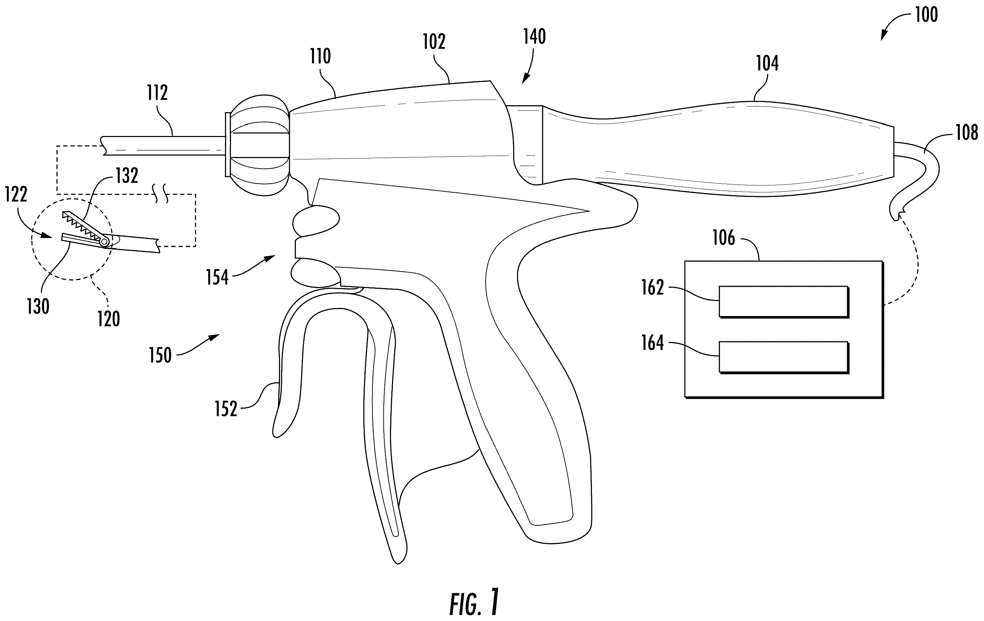

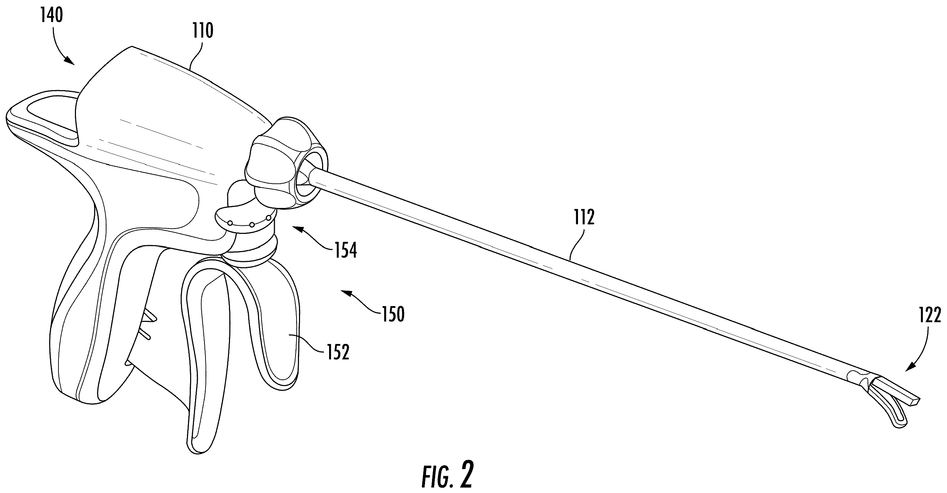

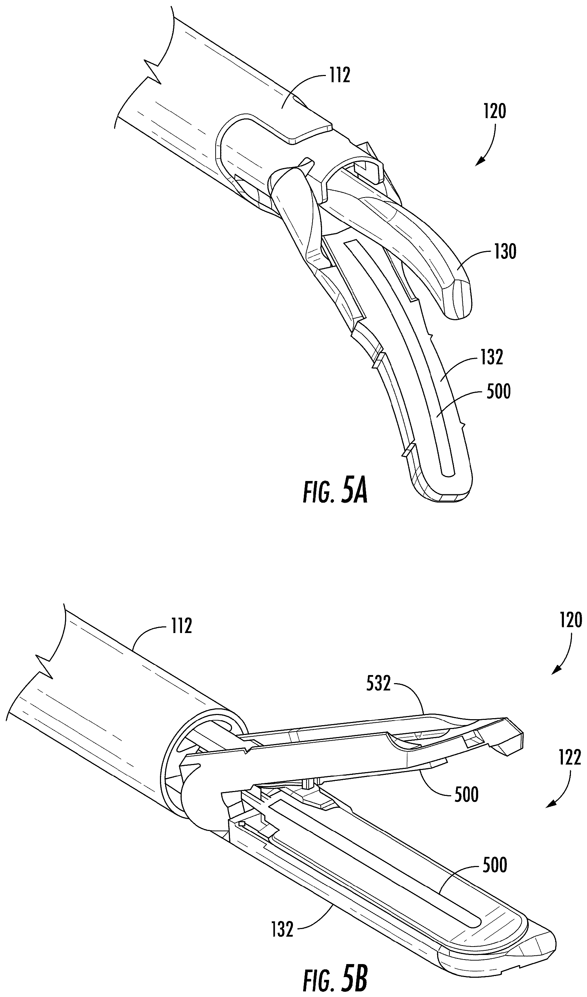

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

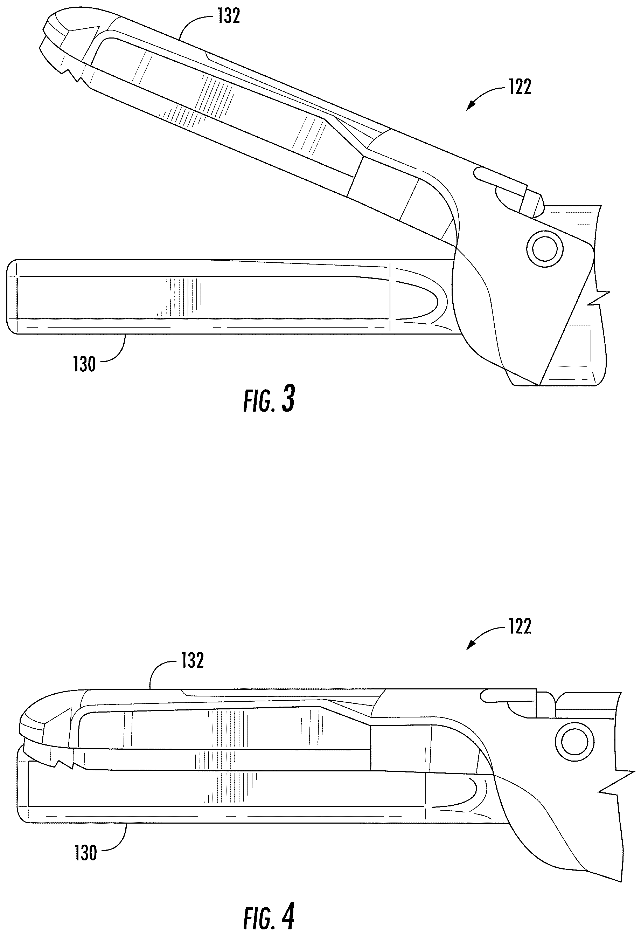

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both an ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

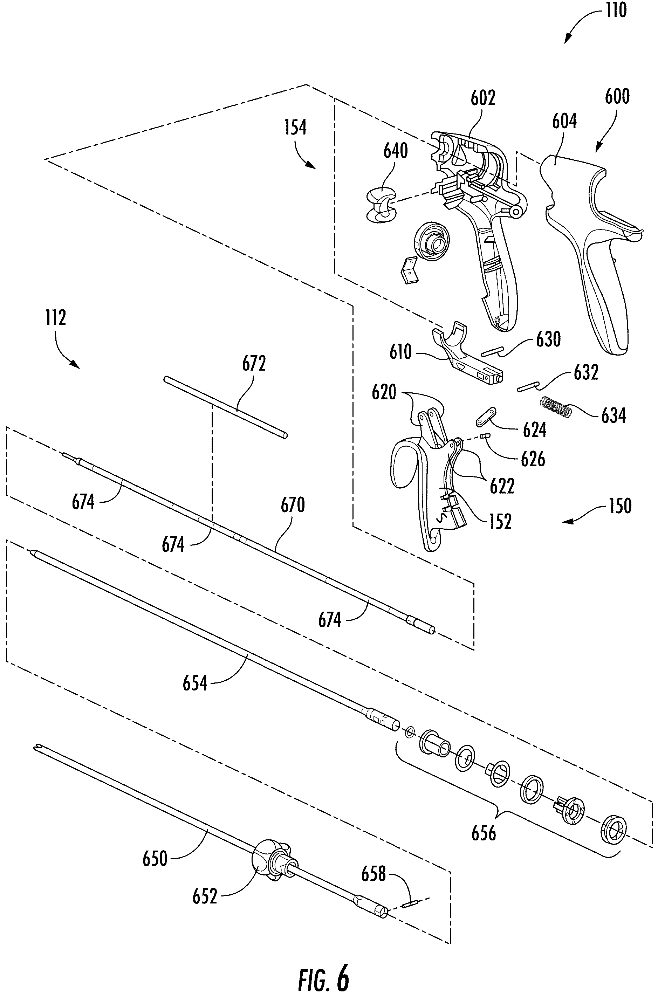

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half-housings 602, 604 are configured to mate with each other to form the housing 600. To facilitate such mating, each of the half-housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

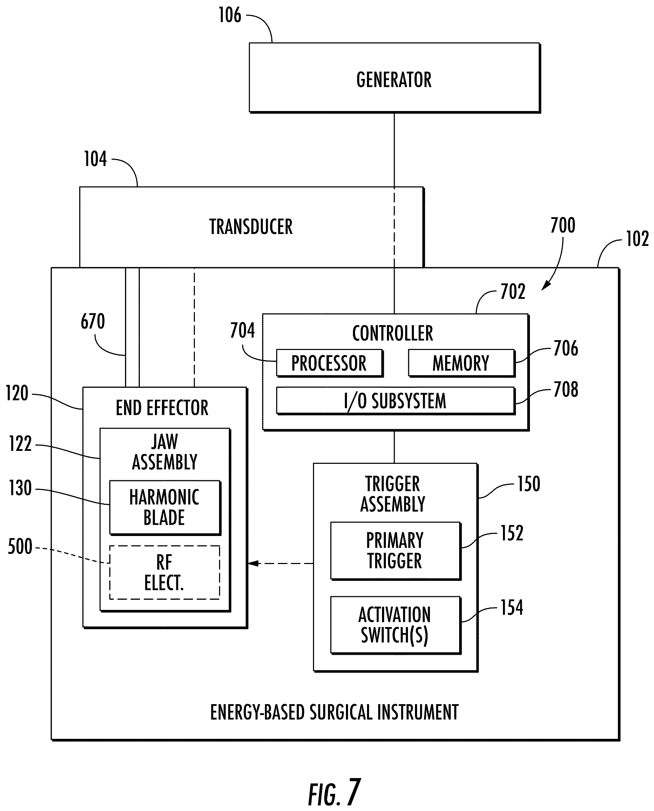

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

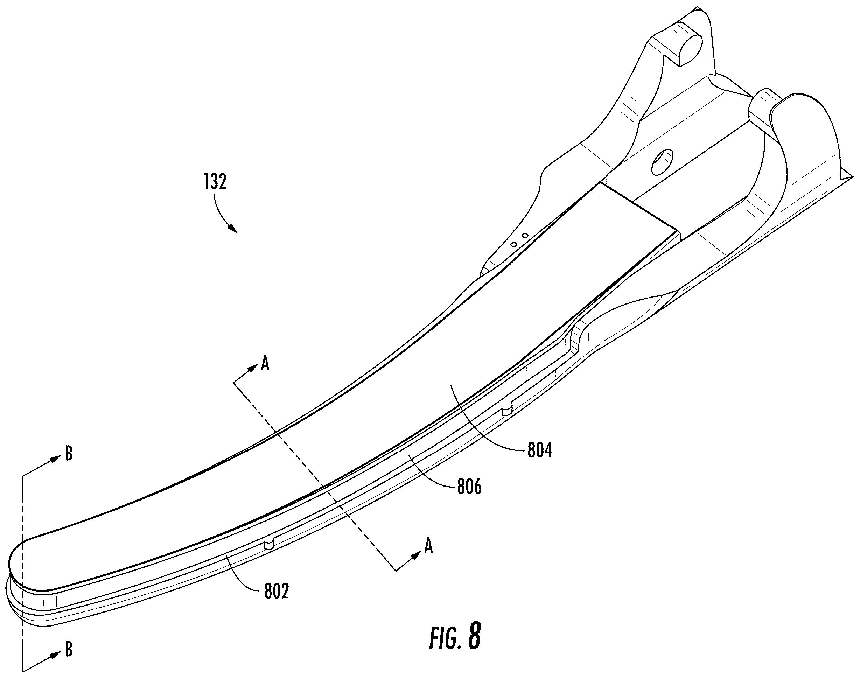

Referring now to FIG. 8, in another embodiment, the jaw clamp 132 is fabricated to include an elongated metallic frame 802 and a non-stick tissue pad 804, which is attached to the frame 802. The tissue pad 804 includes a polymeric substrate 806, which is formed from polytetrafluoroethylene (PTFE). PTFE is a solid fluoropolymer that is chemically inert and has a very low coefficient of friction. Accordingly, the PTFE substrate 806 may exhibit reduced sticking with tissue and other material. In use, when the jaw assembly 122 is actuated from the open state to the closed state, the jaw clamp 132 and thus the tissue pad 804 may contact and otherwise grip tissue and/or blood vessels. In some embodiments, the tissue pad 804 may include one or more tissue stability features such as teeth, posts, ridges, or other features (not shown), which may be formed in the PTFE substrate 806.

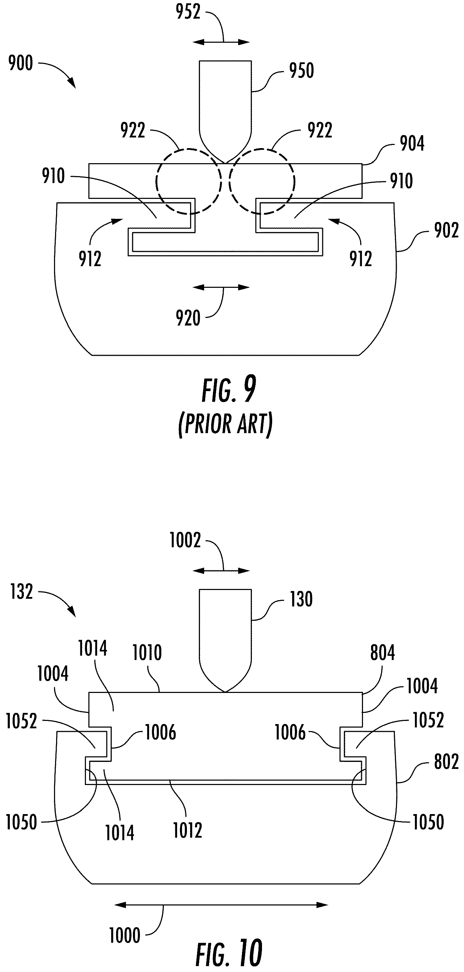

In the illustrative embodiment, the jaw clamp 132 includes retaining features that facilitate the securement of the tissue pad 804 to the metallic frame 802 and reduce the likelihood of the tissue pad 804 splitting under functional load. For example, a cross-sectional view of a typical jaw clamp 800 is shown in FIG. 9. The typical jaw clamp 900 includes a metallic frame 902 and a tissue pad 904 that is retained in the metallic frame 902. The tissue pad 904 is attached to the metallic frame 902 via a pair of tabs 910 which extend inwardly from the frame 902 and which are received in channels or slots 912 of the tissue pad 904. The channels 912 extend inwardly into the sidewalls of the tissue pad 904 to define a minimum width 920 of the tissue pad 904. In the typical jaw clamp 900, the minimum width 902 of the tissue pad 904 is substantially equal or even smaller than a width 952 of an ultrasonic blade 950 used with the jaw clamp 900. As such, in use, the ultrasonic blade 950 may case stress fractures and failures in areas 922 near the channels 912 that define the minimum width 950 of the tissue pad 904 as shown in FIG. 9.

Conversely, as described in more detail below, the illustrative tissue pad 804 has a minimum width 1000 that is greater than a width 1002 of the ultrasonic blade 130 as illustrated in the embodiment of FIG. 10. For example, tissue pad 804 may have a minimum width 1000 that is two times, three times, four times, five times, or greater than the width 1002 of the ultrasonic blade 103. In the illustrative embodiment of FIG. 10, the tissue pad 804 includes a pair of sidewalls 1004, each of which have a channel or slot 1006 defined therein. Each slot 1006 extends longitudinally down the length of the corresponding sidewall 1004. The slots 1006 extend inwardly into the sidewalls 1004 and cooperate to define the minimum width 1000 of the tissue pad 804 (i.e., a width measured between the inner sides of the slots 1006 as shown in FIG. 10), which is greater than the width 1002 of the ultrasonic blade 130 as discussed above.

The illustrative tissue pad 804 also includes a top surface 1010 and a bottom surface 1012 opposite the top surface 1010 and which abuts the metallic frame 802. In the illustrative embodiment, the width of the top surface 1010 is substantially equal to the width of the bottom surface 1012, As such, the top surface 1010 and the bottom surface 1012 each includes tabs 1014 that extend laterally therefrom to define the corresponding slot 1006. Illustratively, the tab 1014 of the top surface 1010 has a greater thickness than the thickness of the tab 1014 of the bottom surface 1012, but may have the same thickness or a lesser thickness in other embodiments.

The metallic frame 802 includes a pair of inner sidewalls 1050, each of includes a tab 1052 that extends laterally inwardly as shown in FIG. 10. The tabs 1052 are received in the slots 1006 of the tissue pad 804 to retain the tissue pad 804 in the metallic frame 802.

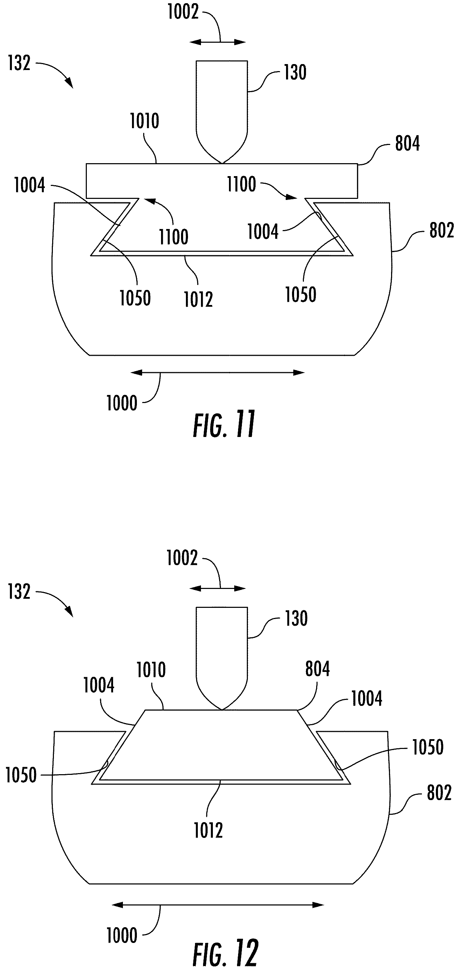

Referring now to FIG. 11 , in another illustrative embodiment, each of the sidewalls 1004 of the tissue pad 804 and the inner sidewalls 1050 of the metallic frame 802 are beveled or otherwise chamfered. In the illustrative embodiment, for example, the sidewalls 1004 of the tissue pad 804 are beveled outwardly, and the sidewalls 1050 of the metallic frame 802 are beveled inwardly (i.e., in a downward or inferior direction). The beveled sidewalls 1004, 1050 cooperate to retain the tissue pad 804 in the metallic frame 802.

In the illustrative embodiment of FIG. 11, each beveled sidewall 1004 of the tissue pad 804 has an innermost point 1100, which is located at the uppermost point of the beveled sidewall, that defines the minimum width 1000 of the tissue pad 804. As discussed above, the minimum width 1000 of the tissue pad 804 is greater than the width 1002 of the ultrasonic blade 130 (e.g., by two, three, four, five times or greater). Additionally, in the illustrative embodiment of FIG. 11, the bottom surface 1012 of the tissue pad 804 may have a width that is substantially equal to, greater than, or slightly less than the width of the top surface 1010.

Referring now to FIG. 12, in another embodiment, the tissue pad 804 may have a substantially trapezoidal cross-sectional shape in some embodiments. In such embodiments, the bottom surface 1012 has a width that is greater than the width of the top surface 1010. Additionally, in such embodiments, the width of the top surface 1010 defines the minimum width 1000 of the tissue pad 804, which is greater than the width of the ultrasonic blade 130 (e.g., by two, three, four, five times or greater).

Similar to the embodiment of FIG. 11, the sidewalls 1004 of the tissue pad 804 are beveled outwardly. Additionally, the sidewalls 1050 of the metallic frame 802 are beveled inwardly (i.e., in a downward or inferior direction). Each of the sidewalls 1004, 1050 are configured to retain the tissue pad 804 in the metallic frame 802.

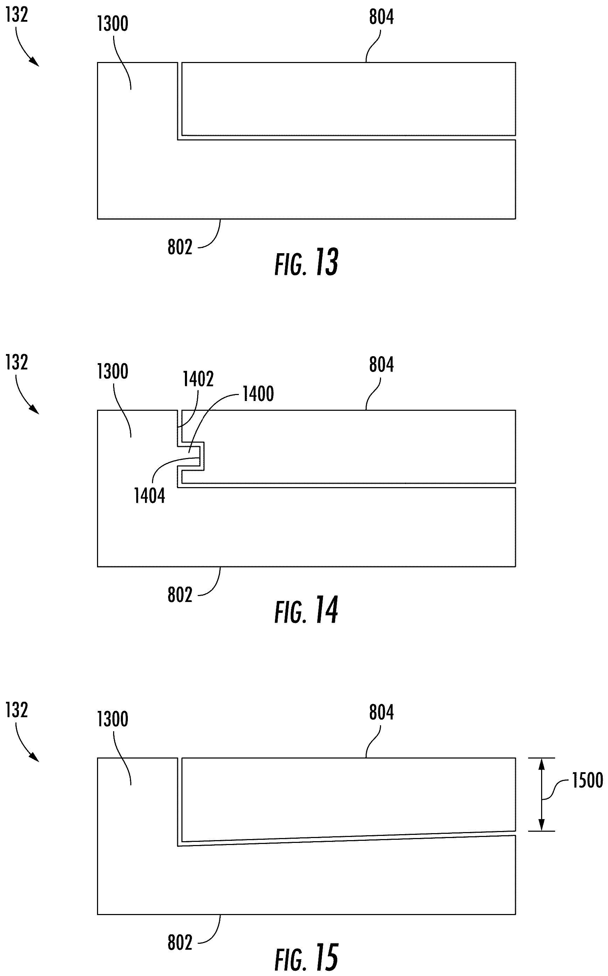

Referring now to FIG. 13, in some embodiments, the metallic frame 802 may include a distal end or nub 1300 that extends upwardly to capture the tissue pad 804. That is, unlike typical tissue pads that extend the full length of the frames of the corresponding jaw clamps, the tissue pad 804 is encapsulated by the distal nub 1300 of the metallic frame 802. The distal nub 1300 is configured to apply an amount of longitudinal force backwardly onto the tissue pad 804 to improve the retainment of the tissue pad in the metallic frame 802. In the illustrative embodiment, the distal nub 1300 extends upwardly approximately equal to the top surface 1010 of the tissue pad 804.

Referring to FIG. 14, in some embodiments, the distal nub 1300 may include a tab 1400 extending proximally from an inner wall 1402. The tab 1400 is received in a corresponding channel 1404 of the tissue pad 804 to further retain the tissue pad 804 in the metallic frame 820.

Additionally, in some embodiments, the thickness 1500 of the tissue pad 804 may not be constant along the longitudinal length of the tissue pad 804. For example, as shown in FIG. 15, the thickness 1500 of the tissue pad 804 may increase toward the distal end of the tissue pad 804. In such embodiments, the metallic frame 802 may have a corresponding increasing recess configured to receive the increasing thickness of the tissue pad 804 as shown in FIG. 15.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. An end effector of a surgical instrument, the end effector comprising:
a jaw clamp including a metallic frame and a tissue pad attached to the metallic frame, wherein the tissue pad comprises a polytetrafluoroethylene (PTFE) substrate; and
an ultrasonic blade configured to contact the tissue pad of the jaw clamp when the end effector is in a closed position,
wherein the metallic frame of the jaw clamp includes a pair of metallic sidewalls and each metallic sidewall includes a tab extending longitudinally along the corresponding metallic sidewall, and
wherein the tissue pad includes a pair of sidewalls and each sidewall includes a slot extending longitudinally along the corresponding sidewall, wherein a minimum width of the tissue pad is defined between the slots of the pair of sidewalls and the minimum width of the tissue pad is greater than a width of the ultrasonic blade.

2. The end effector of claim 1, wherein each sidewall of the tissue pad is a vertical sidewall and the tabs of the metallic sidewall extend laterally into the slots of the sidewalls.

3. The end effector of claim 1 or claim 2, wherein each sidewall of the tissue pad includes an upper tab that extends laterally outwardly from the tissue bad and a lower tab that extends laterally outwardly from the tissue pad to define the respective slot in the sidewall.

4. The end effector of any preceding claim, wherein the metallic frame includes a distal end that wraps upwardly around a distal end of the tissue pad.

5. The end effector of any preceding claim, wherein the tissue pad further includes a top surface that is contacted by the ultrasonic blade and a bottom surface opposite the top surface, wherein each of the top surface and the bottom surface have equal widths.

6. An end effector of a surgical instrument, the end effector comprising:
a jaw clamp including a metallic frame; and
a tissue pad attached to the metallic frame, wherein the tissue pad comprises a polytetrafluoroethylene (PTFE) substrate; and
wherein the metallic frame of the jaw clamp includes a pair of beveled metallic sidewalls, and
wherein the tissue pad includes a pair of beveled sidewalls configured to mate with the beveled sidewalls of the metallic frame to retain the tissue pad in the metallic frame.

7. The end effector of claim 6, further comprising an ultrasonic blade configured to contact the tissue pad when the end effector is in a closed position,
wherein an uppermost point of each beveled sidewall defines a minimum width of the tissue pad, and wherein the minimum width of the tissue pad is greater than a width of the ultrasonic blade.

8. The end effector of claim 7, wherein the tissue pad further includes a top surface that is contacted by the ultrasonic blade and a bottom surface opposite the top surface, wherein each of the top surface and the bottom surface have different widths.

9. The end effector of claim 8, wherein the top surface of the tissue pad has a width that is one of greater than and less than the width of the bottom surface of the tissue pad.

10. The end effector of claim 8 or claim 9, wherein the top surface includes a pair of lateral tabs that extend laterally over the corresponding beveled sidewalls of the tissue pad.

11. A surgical instrument comprising:
an end effector having a jaw clamp and an ultrasonic blade,
wherein the jaw clamp includes a metallic frame and a tissue pad attached to the metallic frame, and
wherein the tissue pad of the jaw clamp has a minimum width that is greater than a width of the ultrasonic blade.

12. The surgical instrument of claim 11, wherein the tissue pad includes a polytetrafluoroethylene (PTFE) substrate.

13. The surgical instrument of claim 11 or claim 12, wherein the metallic frame of the jaw clamp includes a pair of metallic sidewalls and each metallic sidewall includes a tab extending longitudinally along the corresponding metallic sidewall, and
wherein the tissue pad includes a pair of sidewalls and each sidewall includes a slot extending longitudinally along the corresponding sidewall, wherein the tabs of the metallic sidewalls extend into the corresponding slots of the tissue pad.

14. The surgical instrument of any one of claims 11 to 13, wherein the metallic frame includes a pair of beveled sidewalls and the tissue pad includes a pair of beveled sidewalls configured to mate with the beveled sidewalls of the metallic frame to retain the tissue pad in the metallic frame.

15. The end effector of claim 6 or the surgical instrument of claim 14, wherein the beveled sidewalls of the metallic frame are beveled inwardly, and wherein the beveled sidewalls of the metallic frame are beveled outwardly.

16. The end effector of claim 6 or the surgical instrument of claim 11, wherein the metallic frame includes a distal end that wraps upwardly around a distal end of the tissue pad.
